# FASCICULE DE BREVET EUROPEEN

(11) **EP 0 778 350 B1**
(45) Date de publication et mention de la délivrance du brevet: **03.04.2002**
(21) Numéro de dépôt: 95203265.4
(22) Date de dépôt: 28.11.1995
(51) Int. Cl.: C12P 3/00

(54) **Procédé de transformation du soufre élémentaire en sulfure**
Verfahren zur Umwandlung von elementarem Schwefel in Sulfid
Process for the transformation of elementary sulphur to sulfide

(43) Date de publication de la demande: 11.06.1997
(73) Titulaire: SOCIETE DES PRODUITS NESTLE S.A., 1800 Vevey (CH)
(72) Inventeur: Cerny, Christoph, CH-8400 Winterthur (CH); Huynh-Ba, Tuong, CH-1009 Pully (CH); Matthey-Doret, Walter, CH-1092 Belmont S/Lausanne (CH)
(74) Mandataire: Archambault, Jean

(56) Documents cités:
- EP-A- 0 395 556
- US-A- 3 642 497

## Description

La présente invention a pour objet un procédé de biotransformation de soufre élémentaire en sulfure et un procédé de préparation d'un agent aromatisant, notamment produisant un arôme de type viande.

Etant donné le rôle central joué par le sulfure d'hydrogène dans la génération des arômes de type viande, il y a un intérêt à trouver des sources alternatives de soufre générant du sulfure. Une voie particulièrement intéressante est constituée par la fermentation microbienne. On a décrit, par exemple dans Amer. J. Enol. Vitic., 1977, 28 (3), 137-144, Schütz M. et al, la présence de sulfure d'hydrogène responsable de la production de mauvaises odeurs dans les résidus de vinification.

Une autre voie est décrite dans US-A-3 642 497, qui a trait à des réactions entre un pentose et le sulfure d'hydrogène, en milieu neutre ou acide et à une température comprise entre 100°C et 120°C, dans 1e but de produire des compositions ayant des caractéristiques d'arômes de viande.

Nous avons trouvé que certains micro-organismes utilisables en alimentation, notamment la levure boulangère, Saccharomyces cerevisae, avaient la capacité de former du sulfure d'hydrogène à partir de soufre élémentaire par fermentation biologique. Une telle source est particulièrement intéressante dans la mesure où elle est abondante et économique.

Le procédé selon l'invention est caractérisé par le fait que l'on incube un micro-organisme utilisable en alimentation, notamment la levure boulangère, avec du soufre élémentaire en présence d'un sucre réducteur.

L'invention concerne également un procédé de préparation d'un agent aromatisant, caractérisé en ce que l'on incube un bouillon du micro-organisme, notamment de levure boulangère en présence de soufre élémentaire et d'un sucre réducteur à une température où il est actif, ce qui produit un dégagement gazeux, que l'incubation dure jusqu'à cessation du dégagement gazeux, puis que l'on traite thermiquement le milieu réactionnel de manière à développer l'arôme.

Pour mettre en oeuvre le présent procédé, on prépare un mélange contenant le micro-organisme, par exemple une crème ou un extrait de levure boulangère, dont on ajuste le pH, par exemple à 5-9, de préférence à 6-8, au moyen d'une solution tampon. On y ajoute du soufre élémentaire et progressivement un sucre réducteur, sous agitation, de préférence vigoureuse.

Le sucre réducteur peut être, par exemple, un monosaccharide et notamment un pentose tel que le xylose, le ribose ou l'arabinose, ou de préférence un hexose tel que le glucose ou le fructose. On ajoute ce dernier de préférence progressivement, par exemple en 60 à 90 min. environ.

Dès le début de l'incubation, il se produit un dégagement gazeux de sulfure d'hydrogène. Les conditions d'incubation sont choisies pour une activation optimale du micro-organisme, par exemple à une température de 25 à 40 ° C, à un pH de 5 à 9 et pendant 1 à 7 h, sous agitation et ceci jusqu'à cessation du dégagement gazeux.

Après l'incubation, on peut traiter thermiquement le milieux réactionnel, de préférence au reflux à 100° C, pendant 20 à 40 min. C'est par ce traitement que se développe l'arôme recherché dans le surnageant que l'on peut séparer, par exemple par centrifugation, puis le cas échéant concentrer et sécher, par exemple par pulvérisation ou lyophilisation en présence d'un support, par exemple de maltodextrine ou de cyclodextrine.

L'invention concerne également la préparation d'arômes de procédé, pour laquelle le milieu réactionnel contenant l'agent aromatisant ou son précurseur réactif peut être mélangé avec d'autres matières riches en précurseurs et/ou en renforçateurs d'arômes.

Ces autres matières peuvent être, par exemple une sauce de soya, un autolysat de levure, le chlorure de sodium, certains sucres, des graisses, des aromates.

Pour préparer de tels arômes de réaction, on peut faire réagir le mélange par chauffage à 80-150° C, de préférence environ 100° C durant 30 min. à 4 h.

On peut ensuite a jouter au produit de réaction un support, par exemple de la maltodextrine ou de la cyclodextrine, puis sécher le mélange à température modérée, par exemple à 60-70° C sous vide.

De tels arômes peuvent être incorporés dans des aliments destinés à la nourriture humaine ou animale.

Les exemples ci-après illustrent l'invention. Dans ceux-ci, les pourcentages et parties sont en poids, sauf indication contraire.

### Exemples 1-3

On clarifie de la crème de levure boulangère du commerce, Saccharomyces cerevisae à 22-28 % de matière sèche par centrifugation et on élimine le surnageant. On mélange ensuite le résidu avec une solution aqueuse à 0,1 M de tampon phosphate de pH choisi.

On place alors 300 ml de cette crème de cellules entières dans un réacteur en verre équipé d'une sonde de température réglée à 30° C et d'un agitateur tournant à 600 t/min., reliée à un pH-stat maintenant le pH à la valeur choisie par adjonction d'une solution aqueuse 2N d'hydroxyde de sodium et d'un réfrigérant. On y ajoute 0,8 g (25 mmol) de soufre élémentaire colloïdal, puis, goutte à goutte une solution de glucose autoclavé (18 g, 100 mmol, dissous dans 30 ml d'eau), de même pH, cette dernière adjonction durant 90 min.

Dès le début de l'adjonction de glucose on observe un dégagement gazeux spontané qui cesse après 5 h d'incubation. On peut quantifier ce dégagement au moyen d'un flacon piège monté à la sortie du réfrigérant et contenant 60 ml d'une solution aqueuse 0,5 M de nitrate de plomb (II).

Après 6 h de fermentation, on ajuste, le cas échéant, le pH du bouillon à 6 et on recueille le précipité noir de sulfure de plomb formé dans le piège, puis on le sèche en étuve à 100° C pendant 12 h. Les quantités pondérales respectives de sulfure d'hydrogène produites, calculées comme sulfure de plomb ainsi que les rendements en sulfure d'hydrogène sont indiqués dans le tableau 1 ci-après.

**Tableau 1**

| **Exemple, pH** | **1, pH 6** | **2, pH 7** | **3, pH 8** |
|---|---|---|---|
| PbS (mg) | 1420 | 1765 | 2548 |
| PbS (mmol) | 5,94 | 7,38 | 10,7 |
| Rendement en sulfure d'hydrogène (%) | 23,8 | 29,5 | 42,6 |

### Exemple 4

Après incubation selon l'exemple 1, on chauffe le bouillon à 100° C pendant 30 min. Après refroidissement à la température ambiante, on sépare le surnageant par centrifugation pendant 15 min. à 5000 t/min., puis on extrait le résidu à l'eau et on sépare de nouveau le surnageant par centrifugation et on combine les deux surnageants en un seul.

Après avoir ajusté le pH du surnageant à 4 avec une solution aqueuse 2N d'acide chlorhydrique, on le sature avec du chlorure de sodium, puis on l'extrait l'échantillon avec 300 ml d'éther diéthylique au moyen d'un extracteur liquide/liquide, on sépare la phase organique, on la sèche sur sulfate de sodium, on la concentre dans une colonne de Vigreux en un volume de 5 ml et on entrepose la solution concentrée obtenue dans un congélateur à - 40° C jusqu'à l'analyse.

On a analysé l'extrait concentré sur le plan olfactif et chimique. L'analyse par chromatographie gazeuse est effectuée sur chromatographe Carlo Erba Mega 2 équipé d'un injecteur à froid et d'un détecteur d'ionisation de flamme (GC-FID). On utilise un détecteur photométrique de flamme (GC-FPD) pour les composés soufrés. Les colonnes capillaires sont DB-5 et DB-FFAP, 30 m x 0,32 mm, épaisseur de film 0,25 micron, J&W Scientific, Folsom, USA. Le gaz vecteur est l'hélium (65 kPa) avec complément d'azote (40 kPa) pour la GC-FID. L'analyse olfactive est effectuée à l'aide de la technique de chromatographie gazeuse couplée à l'olfactométrie (GC/O). A cette fin, l'effluent gazeux est divisé en deux parties égales. Une moitié va vers le détecteur FID. L'autre moitié s'achemine vers un dispositif où les constituants volatiles sont reniflés et leur qualité odorante décrite par l'analyste. Cette analyse olfactométrique est réalisée par deux analystes. On calcule les indices de rétention (RI) par interpolation linéaire.

Les analyses ont été confirmées par l'établissement de spectres produits par couplage d'un chromatographe gazeux à un spectrographe de masse à impact d'électron (GC/EI-MS, HP 5890/HP 5971) dans les mêmes conditions opératoires que pour GC-FID.

Les résultats pour les composés soufrés principaux identifiés sont indiqués dans le tableau 2 ci-après.

**Tableau 2**

| **Composé** | **RI (FFAP), FID** | **RI (FFAP), FPD** | **RI (FFAP), Sniffing** | **Odeur** | **FFAP-FPD Surface des pics (%)** |
|---|---|---|---|---|---|
| O-Méthyl thioacétate | - | 1005 | 1002 | Fruitée, sucrée, beurrée | 1,87 |
| S-Méthyl thioacétate | 1053 | 1060 | 1056 | Fruitée | 2,02 |
| S-Ethyl thioacétate | 1072 | 1100 | 1085 | Fruitée, sucrée | 0,18 |
| Acide thioacétique | 1107 | 1130 | 1118 | Acidulée, viandée, rôtie | 78,38 |
| S-Méthyl-thio thioacétate | 1464 | 1466 | 1470 | Céréale grillée | 2,33 |
| S-Méthyl-thiométhyl thioacétate | - | 1535 | 1545 | Viandée, bouillon | 1,33 |
| 3-Méthyl-thio-1-propanol | 1718 | 1722 | 1729 | Légume, pomme de terre | 2,87 |

Différents autres composés soufrés non identifiés représentaient 7,9 % de la surface totale des pics et contribuaient à l'odeur de bouillon de légumes/céréales.

On a constaté que l'extrait concentré présentait globalement une forte flaveur rappelant le fromage grillé semblable à celle de la crôute grillée produite dans la fondue au fromage ou la raclette.

### Exemple 5

On utilise le bouillon produit par l'incubation selon l'exemple 1 dans une réaction de production d'arôme de viande en chauffant les ingrédients sauf la maltodextrine dans les proportions indiquées au tableau 3 ci-après pendant 150 min. à 100° C à pH 5. Après la réaction, on ajoute la maltodextrine et sèche le mélange à 65°C sous vide. A titre de comparaison, on réalise la même réaction avec de la levure boulangère n'ayant pas subi d'incubation avec le soufre sans sulfure de sodium ajouté (comparaison 1), avec sulfure de sodium ajouté (comparaison 2) et avec cystéine (à raison de 1 mole de cystéine pour 1/5ème mole de sulfure produit par l'incubation) (comparaison 3).

**Tableau 3**

| **Ingrédients (%)** | **Exemple 5** | **Comparaison 1** | **Comparaison 2** | **Comparaison 3** |
|---|---|---|---|---|
| Sel, extrait d'oignon, sauce soja en poudre | 28,6 | 28,6 | 28,6 | 28 |
| Farine de viande de poulet , graisse de poule | 6,5 | 6,5 | 6,5 | 6,4 |
| Saccharose, xylose, glucose | 6,1 | 6,1 | 6,1 | 6 |
| Extrait de levure | 5,5 | 5,5 | 5,4 | 5,4 |
| Crème de levure incubée avec du soufre | 42,5 | - | - | - |
| Crème de levure incubée sans soufre | - | 42,5 | 42,5 | 42,5 |
| Sulfure de sodium (cristallin à 60 %) | - | - | 0,2 | - |
| Cysteine HCl | - | - | - | 1 |
| Maltodextrine | 10,8 | 10,8 | 10,8 | 10,8 |

A la dégustation par un panel de 5 personnes expérimentées d'un bouillon contenant 6 g d'arôme et 1 g de NaCl dissous dans 500 ml d'eau chaude, l'extrait préparé selon l'invention a montré une flaveur viandée distincte comparé à une note générale de bouillon pour la comparaison 1, cependant que la comparaison 2 posséda une flaveur viandée avec un léger accent soufré. L'arôme de l'extrait préparé selon l'invention est très proche de celui de la comparaison 3.

## Revendications

1. Procédé de biotransformation de soufre élémentaire en sulfure, **caractérisé par le fait que** l'on incube un micro-organisme utilisable en alimentation, notamment la levure boulangère, avec du soufre élémentaire en présence d'un sucre réducteur choisi parmi les monosaccharides et notamment un pentose tel que le xylose ou l'arabinose, ou un hexose tel que le glucose ou le fructose.

2. Procédé selon la revendication 1, **caractérisé par le fait que** l'on prépare un mélange contenant le micro-organisme, notamment une crème ou un extrait de levure boulangère, dont on ajuste le pH à 5-9 au moyen d'une solution tampon, qu'on y ajoute du soufre élémentaire et progressivement le sucre réducteur, sous agitation en 60 à 90 min.

3. Procédé selon la revendication 2, **caractérisé par le fait que** l'incubation a lieu à une température de 25 à 40 ° C, à un pH de 5 à 9 et pendant 1 à 7 h, sous agitation et ceci jusqu'à cessation du dégagement gazeux.

4. Procédé de préparation d'un agent aromatisant, **caractérisé en ce que** l'on incube un bouillon d'un micro-organisme utilisable en alimentation, notamment de levure boulangère en présence de soufre élémentaire et d'un sucre réducteur choisi parmi les monosaccharides à une température où il est actif, ce qui produit un dégagement gazeux, que l'incubation dure jusqu'à cessation du dégagement gazeux, puis que l'on traite thermiquement le milieu réactionnel de manière à développer l'arôme.

5. Procédé selon la revendication 4, **caractérisé par le fait qu'**après l'incubation, on traite le milieu réactionnel au reflux à 100° C, pendant 20 à 40 min., que l'on sépare le surnageant, notamment par centrifugation, puis que, le cas échéant, on le concentre et le sèche, notamment par pulvérisation ou lyophilisation en présence d'un support, notamment de maltodextrine ou de cyclodextrine.

6. Procédé pour la préparation d'arômes de procédé selon la revendication 4 ou 5, **caractérisé par le fait que** l'on mélange le milieu réactionnel contenant l'agent aromatisant ou son précurseur réactif avec d'autres matières riches en précurseurs et/ou en renforçateurs d'arômes, notamment une sauce de soya, un autolysat de levure, le chlorure de sodium, certains sucres, des graisses, des aromates.

7. Procédé selon l'une des revendications 4 à 6, **caractérisé en ce que** l'on fait réagir le milieu réactionnel contenant l'agent aromatisant ou son précurseur réactif, mélangé avec d'autres matières riches en précurseurs et/où en renforçateurs d'arômes, en le chauffant à 80-150° C, notamment à environ 100° C durant 30 min. à 4 h., de manière à permettre la réaction.

8. Procédé selon l'une des revendications 4 à 7, **caractérisé en ce que** l'on ajoute un support au milieu réactionnel contenant l'agent aromatisant ou son précurseur réactif, mélangé avec d'autres matières riches en précurseurs et/où en renforçateurs d'arômes, ce support étant notamment de la maltodextrine ou de la cyclodextrine, puis que l'on sèche le mélange à température modérée, notamment à 60-70° C sous vide.

9. Aliment destiné à l'alimentation humaine ou animale, **caractérisé par le fait qu'**il contient un produit obtenu par le procédé selon la revendication 4 ou 5 ou une composition aromatique le contenant provenant du procédé selon l'une des revendications 6 à 8.

## Patentansprüche

1. Verfahren zur Biotransformation von elementarem Schwefel in Sulfid, **dadurch gekennzeichnet, dass** man einen in Nahrungsmitteln verwendbaren Mikroorganismus, insbesondere Backhefe, mit elementarem Schwefel in Gegenwart eines reduzierenden Zuckers inkubiert, der aus den Monosacchariden und insbesondere einer Pentose, wie Xylose oder Arabinose, oder einer Hexose, wie Glucose oder Fructose, ausgewählt ist.

2. Verfahren nach Anspruch 1, **dadurch gekennzeichnet, dass** man eine den Mikroorganismus enthaltende Mischung, insbesondere eine Backhefecreme oder einen Backhefeextrakt, herstellt, deren bzw. dessen pH-Wert mit Hilfe einer Pufferlösung auf 5-9 eingestellt wird, und dass man ihr elementaren Schwefel und reduzierenden Zucker allmählich unter Rühren in 60 bis 90 min beigibt.

3. Verfahren nach Anspruch 2, **dadurch gekennzeichnet, dass** die Inkubation bei einer Temperatur von 25 bis 40°C, bei einem pH von 5 bis 9 und während 1 bis 7 h unter Rühren stattfindet, und zwar bis zum Aufhören der Gasentwicklung.

4. Verfahren zur Herstellung eines Aromamittels, **dadurch gekennzeichnet, dass** man eine Brühe eines in Nahrungsmitteln verwendbaren Mikroorganismus, insbesondere von Backhefe, in Gegenwart von elementarem Schwefel und eines aus den Monosacchariden ausgewählten reduzierenden Zuckers bei einer Temperatur, bei der er aktiv ist, inkubiert, was eine Gasentwicklung erzeugt, dass die Inkubation bis zum Aufhören der Gasentwicklung dauert und dass man das Reaktionsgemisch thermisch so behandelt, dass das Aroma entwickelt wird.

5. Verfahren nach Anspruch 4, **dadurch gekennzeichnet, dass** man das Reaktionsgemisch nach der Inkubation unter Rückfluss 20 bis 40 min bei 100°C behandelt, dass man den Überstand insbesondere durch Zentrifugieren abtrennt und dass man ihn dann ggf. konzentriert und insbesondere durch Zerstäubung oder Lyophilisation in Gegenwart eines Trägers, insbesondere in Gegenwart von Maltodextrin oder Cyclodextrin, trocknet.

6. Verfahren zur Herstellung von verfahrensgemäßen Aromastoffen nach Anspruch 4 oder 5, **dadurch gekennzeichnet, dass** man das das Aromamittel oder seinen reaktiven Vorläufer enthaltende Reaktionsgemisch mit anderen an Aromavorläufern und/oder -verstärkern reichen Stoffen, insbesondere mit einer Sojasauce, einem Hefeautolysat, Natriumchlorid, gewissen Zuckern, Fetten, Aromastoffen, mischt.

7. Verfahren nach einem der Ansprüche 4 bis 6, **dadurch gekennzeichnet, dass** man das das Aromamittel oder seinen reaktiven Vorläufer enthaltende Reaktionsgemisch, das mit anderen an Aromavorläufern und/oder -verstärkern reichen Stoffen gemischt ist, reagieren lässt, indem man es auf 80-150°C, insbesondere auf etwa 100°C, während 30 min bis 4 h erhitzt, so dass es reagiert.

8. Verfahren nach einem der Ansprüche 4 bis 7, **dadurch gekennzeichnet, dass** man dem das Aromamittel oder seinen reaktiven Vorläufer enthaltenden Reaktionsgemisch, das mit anderen an Aromavorläufern und/oder -verstärkern reichen Stoffen gemischt ist, einen Träger beigibt, der insbesondere Maltodextrin oder Cyclodextrin ist, und dass man dann die Mischung bei mäßiger Temperatur, insbesondere bei 60-70°C, unter Unterdruck trocknet.

9. Nahrungsmittel zur Ernährung von Menschen oder Tieren, **dadurch gekennzeichnet, dass** es ein in dem Verfahren nach Anspruch 4 oder 5 hergestelltes Produkt oder eine dieses Produkt enthaltende aromatische Zusammensetzung enthält, die aus dem Verfahren nach einem der Ansprüche 6 bis 8 stammt.

## Claims

1. Method for biotransforming elemental sulfur into sulfide, **characterized in that** a food-grade microorganism, especially baker's yeast, is incubated with elemental sulfur in the presence of a reducing sugar chosen from monosaccharides and especially a pentose such as xylose or arabinose, or a hexose such as glucose or fructose.

2. Method according to Claim 1, **characterized in that** a mixture containing the microorganism is prepared, especially a cream or an extract of baker's yeast, the pH of which is adjusted to 5-9 using a buffer solution, and **in that** elemental sulfur is added and, gradually, the reducing sugar, with stirring over 60 to 90 minutes.

3. Method according to Claim 2, **characterized in that** the incubation takes place at a temperature of from 25 to 40°C, at a pH of from 5 to 9 and for 1 to 7 hours, with stirring and until the evolution of gas has ceased.

4. Method for preparing a flavouring, **characterized in that** a broth of a food-grade microorganism, especially of baker's yeast, is incubated in the presence of elemental sulfur and a reducing sugar chosen from monosaccharides, at a temperature at which it is active, which produces an evolution of gas, **in that** the incubation lasts until the evolution of gas has ceased, and **in that** the reaction medium is then heat-treated so as to develop the flavour.

5. Method according to Claim 4, **characterized in that**, after the incubation, the reaction medium is refluxed at 100°C for 20 to 40 minutes, **in that** the supernatant is separated out, especially by centrifugation, and, where appropriate, **in that** it is then concentrated and dried, especially by spraying or freeze-drying in the presence of a support, especially of maltodextrin or of cyclodextrin.

6. Method for preparing process flavours according to Claim 4 or 5, **characterized in that** the reaction medium containing the flavouring or its reactive precursor is mixed with other materials rich in flavour precursors and/or flavour enhancers, especially a soy sauce, a yeast autolysate, sodium chloride, certain sugars, fats and seasonings.

7. Method according to one of Claims 4 to 6, **characterized in that** the reaction medium containing the flavouring or its reactive precursor, mixed with other materials rich in flavour precursors and/or flavour enhancers, is reacted by heating it at 80-150°C and especially at about 100°C for 30 minutes to 4 hours, so as to allow the reaction.

8. Method according to one of Claims 4 to 7, **characterized in that** a support is added to the reaction medium containing the flavouring or its reactive precursor, mixed with other materials rich in flavour precursors and/or flavour enhancers, this support especially being maltodextrin or cyclodextrin, and **in that** the mixture is then dried at moderate temperature, especially at 60-70°C under vacuum.

9. Food intended for human or animal consumption, **characterized in that** it contains a product obtained by the method according to Claim 4 or 5 or a flavouring composition containing it derived from the method according to one of Claims 6 to 8.
